# EUROPEAN PATENT SPECIFICATION

(11) **EP 0 865 785 B1**
(45) Date of publication and mention of the grant of the patent: **14.05.2003**
(21) Application number: 98103184.2
(22) Date of filing: 24.02.1998
(51) Int. Cl.: A61K 7/16, A61K 49/00, A61K 7/22

(54) **Antimicrobial caries-detecting composition**
Antimikrobielle Karies anzeigende Zusammensetzung
Composition antimicrobienne détectrice de caries

(30) Priority: 24.02.1997 JP 3868197; 28.02.1997 JP 4563497
(43) Date of publication of application: 23.09.1998
(73) Proprietor: KURARAY CO., LTD., Kurashiki-City (JP)
(72) Inventor: Fukunishi, Kyoko, Kurashiki-City (JP); Hino, Kenichi, Kurashiki-City (JP)
(74) Representative: Müller-Boré & Partner Patentanwälte

(56) References cited:
- EP-A- 0 363 748
- US-A- 3 332 743
- J. JENSEN: "Binding of dyes to chlorhexidine-treated hydroxyapatite" SCANDINAVIAN JOURNAL OF DENTAL RESEARCH, vol. 85, no. 5, 1977, pages 334-340, XP000870194
- J. JENSEN : "Binding of dyes to hydroxyapatite treated with cetylpyridinium chloride or cetrimonium bromide" SCANDINAVIAN JOURNAL OF DENTAL RESEARCH, vol. 86, no. 2, 1978, pages 87-92, XP000870193
- G. ANSARI ET AL.: "Caries detector dyes-an in vitro assessment of some new compounds" JOURNAL OF ORAL REHABILITATION, vol. 26, no. 6, 1999, pages 453-458, XP000870292

## Description

The present invention relates to an antimicrobial caries-detecting composition for dental use in odontological treatment of caries-infected teeth, with which the part of a tooth as infected with cariogenic microbes is selectively stained prior to removing the infected dentin of the tooth, to facilitate the removal of the infected part, and the present invention relates in more detail to the composition being capable of sterilizing and staining the infected part of teeth at the same time. Using the antimicrobial caries-detecting composition of the invention, the caries-infected dentin can be removed while preventing the cavity and around it from being contaminated with highly-contaminated tissue pieces that may be scattered during the removal of the infected part. Accordingly, providing the antimicrobial caries-detecting composition, the invention provides safer treatment of caries with neither failed removal of the infected dentin nor re-contamination of the treated tooth with the removed infected dentin.

In conventional treatment of tooth caries for removing the infected dentin with cariogenic microbes, the infected part is selectively stained so as to clearly differentiate it from the other parts thereby removing the infected dentin as completely as possible. For that purpose, Japanese Patent Application (JPA) Kokai No. Sho-51-38428 has proposed "caries-detecting composition" comprising a basic fuchsine and a mono- or polyalcohol, and has produced some results. However, even after such a caries-detecting composition has been developed, commercialized and widely used, there is still no end to the report of pulp irritation after caries treatment, which is a serious problem in the field of dentistry.

On the other hand, for prevention of caries, JPA Kokai No. Sho-51-38427 has proposed "dental plaque or calculus-detecting composition", which is to stain dental plaque or the like that adheres onto the tooth surface to cause caries, and JPA Kokai No. Sho-56-96700 has proposed "caries activity-indicating composition", which indicates sufficient daily teeth brushing and with which the dangerous degree of dental plaque is evaluated. The former composition comprises a dye as dissolved in a polyalcohol and/or water, while the latter composition comprises a pH indicator as dissolved in an aqueous solution of a water-soluble polymer to which is added an antibiotic or preservative selected from chloramphenicol compounds and sodium azide compounds.

For the cause of the dental pulp irritation that has been discussed in those proposals, the greatest probability is that the restorative component will penetrate into dental pulp, and various studies have been made on the penetration in question. However, even if the restorative component itself is embedded adjacent to dental pulp, this does not cause any serious irritation. Recently, therefore, a theory of bacterial cariogenesis is being considered significant for the cause of the dental pulp irritation, which is such that the secondary invasion of oral bacteria into cavity and the failed removal of the cariogenic microbes-infected part cause the dental pulp irritation. The recent studies for the prevention of secondary invasion of oral bacteria into cavity are directed to the increase in the adhesiveness between the dental pulp and the restorative material applied thereto to improve the sealing the margin of the restorative material. As a result, recently, the technique of adhering the restorative material to the dentin has reached an extremely high level. Accordingly, cases of dental pulp irritation have decreased in those days, but it could not be said that there has been no report of dental pulp irritation after caries treatment.

The cause of the dental pulp irritation that is left to the last is the failed removal of the caries-infected dentin and the contamination of cleaned cavity with the caries-infected tissue pieces that has been once removed just before the application of a restorative material thereto. Having taken those problems into consideration, the present inventors have assiduously studied various materials capable of exterminating the probabilities of the failed removal of the cariogenic microbes-infected part from teeth and/or the contamination by the once-removed infected dentin to the cleaned cavity and have obtained the following findings.
① A caries-detecting composition could not completely penetrate into the deepest portion of the caries-infected part of teeth by one application, if the infected part is thick. Therefore, a dentist repeatedly applies the caries-detecting composition to the affected area of a tooth to confirm the infected part, and bores a cavity in the affected part. However, if the dentist has failed to finally stain the infected part for the last confirmation, there is a probability that the cariogenic microbes could not be still removed completely.
② At the point at which the removal of the infected dentin has been almost finished, it is often difficult to determine as to whether or not the dentin had been stained, so that the removal of the infected dentin is often incomplete. In case that the cavity bottom is too thin, the cutting tool would often penetrate the dental pulp. In order to evade this trouble, the operating dentist would often stop the removal of the seemingly slightly stained part.
③ A so-called smear layer comprising powdered dentin is inevitably formed on the surface of the dentin from which the infected part has been removed, and there is a probability that bacteria will remain in the smear layer.
④ As dentinal tubules are in the surface of the dentin drilled for the removal of the infected part, the smear layer is pressed into those dentinal tubules to form a smear plug. It is said that the smear plug remained in the opening of dentinal tubules, have an important roll to protect dental pulp from the components of restorative materials.
   However, there is a probability that the smear plug may be infected with cariogenic microbes.
⑤ There is a probability that the highly-contaminated dentin pieces are scattered around the infected tooth during the removal of the infected part and they will be mixed with body fluid such as saliva, thereby will contaminate again the affected part.

No attempt has heretofore been made against the probability of above ⑤.

The failed removal of the infected dentin must be evaded. However, even if the infected dentin could not be removed completely or even if the infected dentin contaminated the affected part, such should not cause cariogenesis if the bacteria existing in the infected dentin are inactivated. For these, if the cavity formed in an infected tooth is treated with a microbicidal solution effective against cariogenic microbes, the intended object shall be attained. Some reports for this trial have been often introduced in various meetings of the dental society and in various odontological journals. However, even if carious cavities are treated with such a microbicidal solution, the matter as to whether or not the microbicidal solution could penetrate throughout the entire region of the infected dentin depends on the thickness of the infected dentin. The pulp cavity has its own inner pressure, and the inner fluid in dentinal tubules flows from the pulp to the surface of the tooth. Therefore, even if a microbicide is applied to the surface of the infected dentin, it is in fact difficult for the microbicide to effectively penetrate into the inner depth of the infected dentin. Accordingly, if the infected dentin is thick, there is a probability that the microbicide applied thereto is often ineffective.

After having been repeatedly studied so as to solve the above-mentioned problems, the present inventors have reached the following points of view.
① Where a caries-detecting composition is used to stain the infected dentin with simultaneously sterilizing it, the powdered dentin formed during a subsequent step of removing the dentin is under sterilized condition. Therefore, the danger of dental pulp irritation due to the contamination of the powdered dentin to the affected part is greatly reduced, and even the danger of contamination of the adjacent teeth and the gums and further the tools that may induce additional cariogenesis is also greatly reduced.
② Where the infected dentin is removed while being simultaneously stained for caries detection and sterilized, the infected dentin is well sterilized with reducing its thickness. In this, therefore, even the bacteria existing in the depth of the affected part can be effectively killed. In particular, after the last staining for the final confirmation, there exist no infected dentin in the cavity. Even if some bacteria have re-adhered to the affected part, they shall be completely free from bacteria. In these conditions, therefore, satisfactory treatment is ensured. On the basis of those findings, the present inventors have further studied the following matters in order to find out an antimicrobial caries-detecting composition.

The microbicide usable in the invention may be any and every known one that has heretofore been used in detergents and microbicidal compositions for dental use. However, ① the preferred are microbicides capable of killing 99 % or more of 10 thousands bacteria per 1 cm³ with a solution having a microbicidal concentration of 1000 µg/ml for 10 seconds, wherein said bacteria includes *Mutans streptococci* and *Lactobacilli*, which are said to be cariogenic bacteria, *Streptococcus mitis*, and *Actinomyces viscosus*, which are said to be bacteria that may have cariogenicity, and strictly anaerobic bacterias which are said to cause dental pulp irritation.
② The microbicides must be soluble in water and/or water-miscible solvents.
③ When stored in the composition of the invention, the microbicides must be stable for a long period of time, and must not discolor the dye to be essential in the composition.
Among conventional microbicides, preferred *are* cationic microbicides, biguanide-type microbicides, halogenated diphenyl ethers and their analogous compounds, as the microbicides for use in the invention. Of those, more preferred is at least one selected from the group consisting of cetylpyridinium hydrochloride, chlorhexidine, trichlosan and irgasan, or a mixture of two or more of them.

More preferably, the caries-detecting composition of the invention still has some additional activities even in the stage of treatment after the removal of the caries-affected part. Specifically, a polymerizable antimicrobial compound having both an antimicrobial group and a polymerizable group in the molecule does not interfere with the subsequent application of a dental adhesive to the surface of the tooth, even though it remains on the surface of the dentin, and, in addition, it copolymerizes with the adhesive to reinforce the adhesive layer during the restorative operation. Being different from the other antimicrobial compounds that do not polymerize but remain as they are, the polymerizable antimicrobial compound remains in the restorative material with being chemically bonded to the material after the dental treatment. Considering the adhesion durability, therefore, the polymerizable antimicrobial compound is expected to exhibit remarkably safe effects. In addition, the compound of that type is further expected to modify the surface of the dentin to be more compatible with dental adhesives. The present inventors have found that, as the antimicrobial compound of that type, preferred are one or more of polymerizable antimicrobial compounds having a (meth)acrylcyl or styrene group and a quaternary ammonium salt group in the molecule and represented by formula 1 through formula 4, and have completed the present invention.

The invention provides an antimicrobial caries-detecting composition, which comprises, water and/or a water-miscible solvent, a dye in a concentration from 0.1 to 2% by weight being capable of staining the caries-infected part of teeth, and at least an antimicrobial agent selected from the group consisting of cationic microbicides, biguanide-type microbicides and halogenated diphenyl ether-type microbicides.

The invention also provides an antimicrobial caries-detecting composition, which comprises water and/or a water-miscible solvent, a dye in a concentration from 0.1 to 2% by weight being capable of staining the caries-infected part of teeth, and at least one polymerizable antimicrobial agent selected from the group consisting of compounds having a (meth)acryloyl or styrene group and a quaternary ammonium salt group in the molecule and represented by the following general formulae 1, 2, 3 and 4:

H₂C=C(R¹)-C(O)-X-R²-Y (1)

wherein
R¹= H or CH₃,
R²= alkylene group of C₂-C₁₂,
= O, S, or NH,
Y = 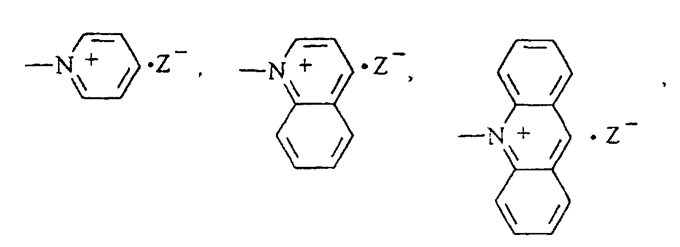 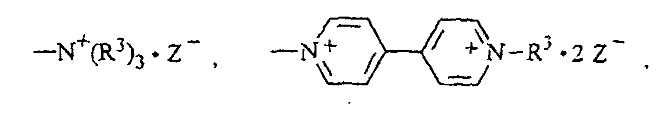 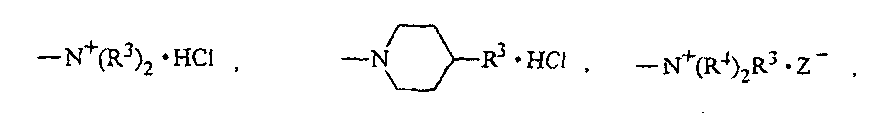 or 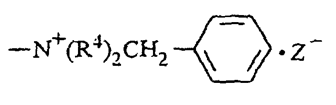 wherein
R³ = H or alkyl group of C₁-C₁₈;
R⁴ = CH₃, CH₂CH₃, or CH₂CH₂OH;
Z = F, Cl, Br, or I;

[H₂C=C(R⁵)-C(O)-X-R⁶-]₂-Y' (2)

wherein
R⁵ = H or CH₃,
R⁶ = alkylene group of C₂-C₁₂,
X = O, S, or NH,
Y' = 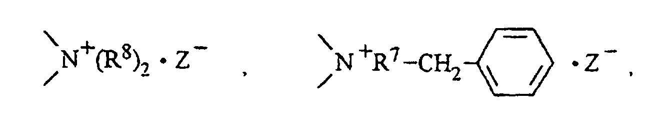 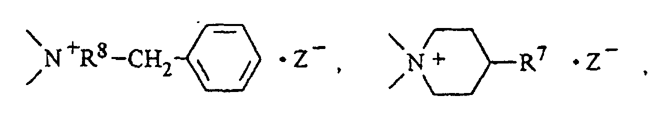 or 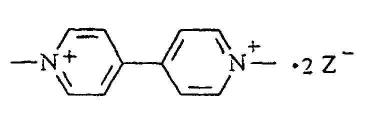 wherein
R⁷ = H or alkyl group of C₁-C₁₈;
R⁸ = CH₃, CH₂CH₃, or CH₂CH₂OH;
Z = F, Cl, Br, or I;

H₂C=C(R⁹)-C₆H₄-R¹⁰-Y (3)

wherein
R⁹ = H or CH₃,
R¹⁰ = alkylene group of C₂-C₁₂,
Y = the same as Y in formula (1); 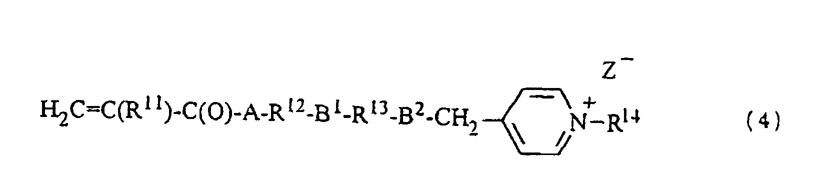 wherein
R¹¹ = H or CH₃,
R¹² = alkylene group of C₁-C₁₂,
R¹³ = alkylene group of C₁-C₁₂,
R¹⁴ = alkyl group of C₁₂-C₂₂,
A = O, S, or NH,
B¹, B² = the same or different groups selected from -CO-, -CO₂-, -O-, -S-, -CCONH- and -NHCO₂-,
Z = F, Cl, Br, or I.

As examples of the microbicides usable in the antimicrobial caries-detecting composition of the invention, include cationic microbicides of various quaternary ammonium salts, of which is preferred cetylpyridinium hydrochloride. Also preferred are biguanide-type microbicides, of which more preferred are various salts of chlorhexidine. Other preferred are halogenated diphenyl ethers and their analogous compounds, of which especially preferred are trichlosan, irgasan. hexachlorophene, etc. As other microbicides usable in the invention, include thymol, clove oil, homosulfamine, nitrofurazone, sulfonamide preparations, nitrofurazone derivatives, acridine-type dyes, formalin preparations, alexidine, cetapron, metafen, sulfonamide, etc. Among those, especially preferred are trichlosan, irgasan, chlorhexidine and cetylpyridinium hydrochloride.

Examples of the polymerizable antimicrobial agents for use in the invention, include quaternary ammonium salts having a (meth)acryloyl group or a styrene group, as those of formula 1, and polymerizable antimicrobial compounds having a (meth)acryloyloxy group and a pyridinium salt group, as those of formula 2. The term, quaternary ammonium salt as referred to herein includes quaternary ammonium salts in the narrow sense and pyridinium salts.

Of those, especially preferred are methacryloyloxydodecylpyridinium bromide, methacryloyloxyhexadecylpyridinium bromide, methacryloyloxyocta decylpyridinium bromide, methacryloyloxydodecylpyridinium chloride, methacryloyloxyhexadecylpyridinium chloride, methacryloyloxyoct adecylpyridinium chloride, N,N-dimethacyloyloxyethyllaurylbenzylammonium bromide, N,N-dimethacyloyloxyethyllaurylbenzylammonium chloride, methacryloyloxyethyl[4-N-octadecylpyridinylmethyl] succinate bromide, methacryloyloxyethyl[4-N-octadecylpyridinylmethyl] succinate chloride, methacryloyloxyethyl[4-N-hexadecylpyridinylmethyl] succinate bromide, methacryloyloxyethyl[4-N-hexadecylpyridinylmethyl] succinate chloride, methacryloyloxyethyl[4-N-dodecylpyridinylmethyl] succinate bromide, methacryloyloxyethyl[4-N-dodecylpyridinylmethyl] succinate chloride, hexadecyl[4-3-(5-methacryloyloxy)valeroyloxy)propyl]pyridinium bromide, hexadecyl[4-(12-methacryloylamino)dodecanoyloxymethyl]pyridinium chloride, etc.

Also usable are 4-vinylbenzylmethyldodecylammonium chloride, 4-vinylbenzylmethylhexadecylammonium chloride, 2-styrylethylmethyldodecylammonium chloride, 2-styrylethylmethylhexadecylammonium chloride, etc.

However, antibiotics of some types, sodium azide, phenol, cresol, hydrogen peroxide, iodoform, hypochlorous acid and the like microbicides are unsuitable for the composition of the invention, since they easily decompose in the composition or will decompose or denature the dye existing in the composition, and, when they remain in the surface of the dentin, they will probably interfere with the subsequent polymerization of the radical-polymerizable composition.

The dye to be used in the antimicrobial caries-detecting composition of the invention must be soluble in the solvent(s), and is capable of visually indicating the part into which the composition has penetrated. In addition, the dye must be those that are not removed from the penetrated part, even when the part is rinsed with water. Preferably, the color of the dye is clearly differentiated from that of the natural dentin, and is capable of forming a striking contrast between its color and the color of the natural dentin. Accordingly, preferred are deep-color dyes such as red, blue, violet or black dyes. Examples of those dyes, include basic fuchsine, eosine, erythrosine, acidic fuchsine, safranine, rose bengale, Phloxine BK, acid red, fast acid magenta, Phloxine B, Fast Green FCF, Rhodamine B, gentian violet, sodium copper chlorophyll, laccaic acid, cochineal, and shisonin. One or more of those dyes are usable in the invention.

The dye concentration in the composition is from 0.1 to 2 % by weight, preferably from 0.1 to 1 % by weight. If it is lower than the defined range, the dye could not satisfactorily stain the intended region. However, if it is higher than the defined range, the dye would stain even the second decalcified layer and even further the healthy area. If so, the determination of the infected area would be difficult.

The solvent to be used in the composition of the invention must be so selected that not only it dissolves the dye and the microbicide in the composition but also it reduces the viscosity of the composition to facilitate the penetration of the composition into the infected dentin. As the solvent, preferred is a water-miscible solvent which is easily miscible with water in any ratios to give a uniform solution. Desirably, the water-miscible solvent compound has not more than 10 carbon atoms, has a polar group, and has a viscosity of not larger than 20 cps. Especially preferred are organic, mono-, di- or tri-hydroxy compounds having from 2 to 10 carbon atoms, as enhancing the penetration of the dye into the caries area to promote the clear coloration of the caries area.

Examples of those compounds are ethanol, ethylene glycol, n-propanol, isopropanol, 1,2-propylene glycol, 1,3-propylene glycol, 1,2-butanediol, 1,3-butanediol, 1,4-butanediol, isobutyl alcohol, n-amyl alcohol, isoamyl alcohol, diethylene glycol, triethylene glycol, tetraethylene glycol, ethylene glycol monomethyl ether, ethylene glycol monoethyl ether, ethylene glycol monobutyl ether, ethylene glycol monoacetate, diethylene glycol monoethyl ether, diethylene glycol monobutyl ether, diethylene glycol monoacetate, triethylene glycol monomethyl ether, triethylene glycol monoethyl ether, glycerin, etc. Of those, preferred are propylene glycol and triethylene glycol, as producing good results.

As other organic solvents also usable in the invention include tetrahydrofuran, dimethylformamide, dimethylsulfoxide, dioxane, acetone, dimethoxyethane. Phenol cannot be used in the invention, as being too toxic. Organic amines, despite of their good ability to fix dyes, are unsuitable for the invention, since they give an offensive odor and they are harmful to human bodies. Hydrocarbons are also unsuitable, since they poorly dissolve dyes and their ability to fix dyes is much inferior to the dye-fixing ability of water.

As has been mentioned hereinabove, these organic solvents may be used in combination with water, and one or more of them may be used in mixture. The blending ratio of the solvents may be suitably determined, depending on the type of the solvents.

The composition of the invention can be prepared easily. For example, a predetermined amount of the dye and a predetermined amount of the microbicide may be added to the organic solvent, distilled water or a mixture thereof, and stirred and dissolved them at room temperature or under mild heating. Alternatively, an excessive amount of the dye and an excessive amount of the microbicide are previously dissolved in the organic solvent, distilled water or a mixture thereof, and the organic solvent, distilled water or their mixture is added to the resulting solution thereby to dilute it to a desired concentration just before its use. Depending on the combination of the microbicide and the solvent, the microbicide may be difficult to dissolve in the solvent. In such a case, any known conventional surfactant may be added to the mixture to give a uniform composition.

To apply the antimicrobial caries-detecting composition of the invention to the cavity of the caries-infected tooth of a patient, the composition is put into a container equipped with a thin long nozzle, and a small amount of the composition is dropped onto the cavity through the nozzle. One to 10 seconds after this application, the cavity is rinsed with water. By this simple operation, just the remaining first decalcified layer is clearly stained with the composition, while the second decalcified layer and the ncn-infected dentin are hardly stained therewith. As a result, the part as infected with cariogenic microbes can be accurately detected out, while, at the same time, the bacteria existing in the infected area are killed or inactivated.

The invention is further described with reference to the following Example.

Various types of dyes, antimicrobial agents and organic solvents usable in the invention are mixed and stirred at room temperature to prepare various detecting ccompositions as in Table 1. These were applied to the cross section of caries-infected teeth extracted from patients, and tested for their stain differentiability and microbicidability.

For the stain differentiability, each sample was applied to the cross section of the caries-infected tooth. About 5 seconds after the application, the applied area was rinsed with water and observed as to whether or not the caries-infected part could be clearly differentiated from the healthy dentin part on the basis of the intrinsic color difference and the difference in hardness between the two parts. From those data, the samples were evaluated in accordance with the following criteria:
++: The caries-infected part was clearly stained.
+ : The caries-infected part was stained.
+-: The caries-infected part was slightly stained.
- : The caries-infected part was hardly stained.

For the microbicidability, each sample was applied to the caries-infected part of the tooth. Ten seconds after the application, the tooth was rinsed with water, and the stained part and the part deeper than the stained part were ground with a carbide bar attached to an air turbine. The resulting powdered teeth were put into a germ-free BHI (brain heart infusion) broth medium, then were treated with ultrasonic vibrations for 10 minutes, and incubated therein overnight. The growth of the cariogenic bacteria was obtained from the light absorbance of the resulting culture. From the data thus obtained, the samples were evaluated in accordance with the following criteria:
++: The growth of bacteria in the stained powdered teeth was inhibited, and no bacteria grew in the non-stained teeth.
+-: The growth of bacteria in the stained powdered teeth was inhibited, but bacteria grew in the non-stained teeth.
--: The growth of bacteria in the stained powdered teeth was not inhibited at all, and bacteria grew in the non-stained teeth.

### Advantages of the Invention

The antimicrobial caries-detecting composition of the invention is effectively used in dental treatment of caries for removing cariogenic microbes that may be left in the cavity of caries-infected teeth. Even if some cariogenic microbes be left in the cavity, the antimicrobial caries-detecting composition can completely kill the remaining microbes thereby effectively inhibiting the dental pulp irritation and the secondary caries to be caused by the remaining microbes.

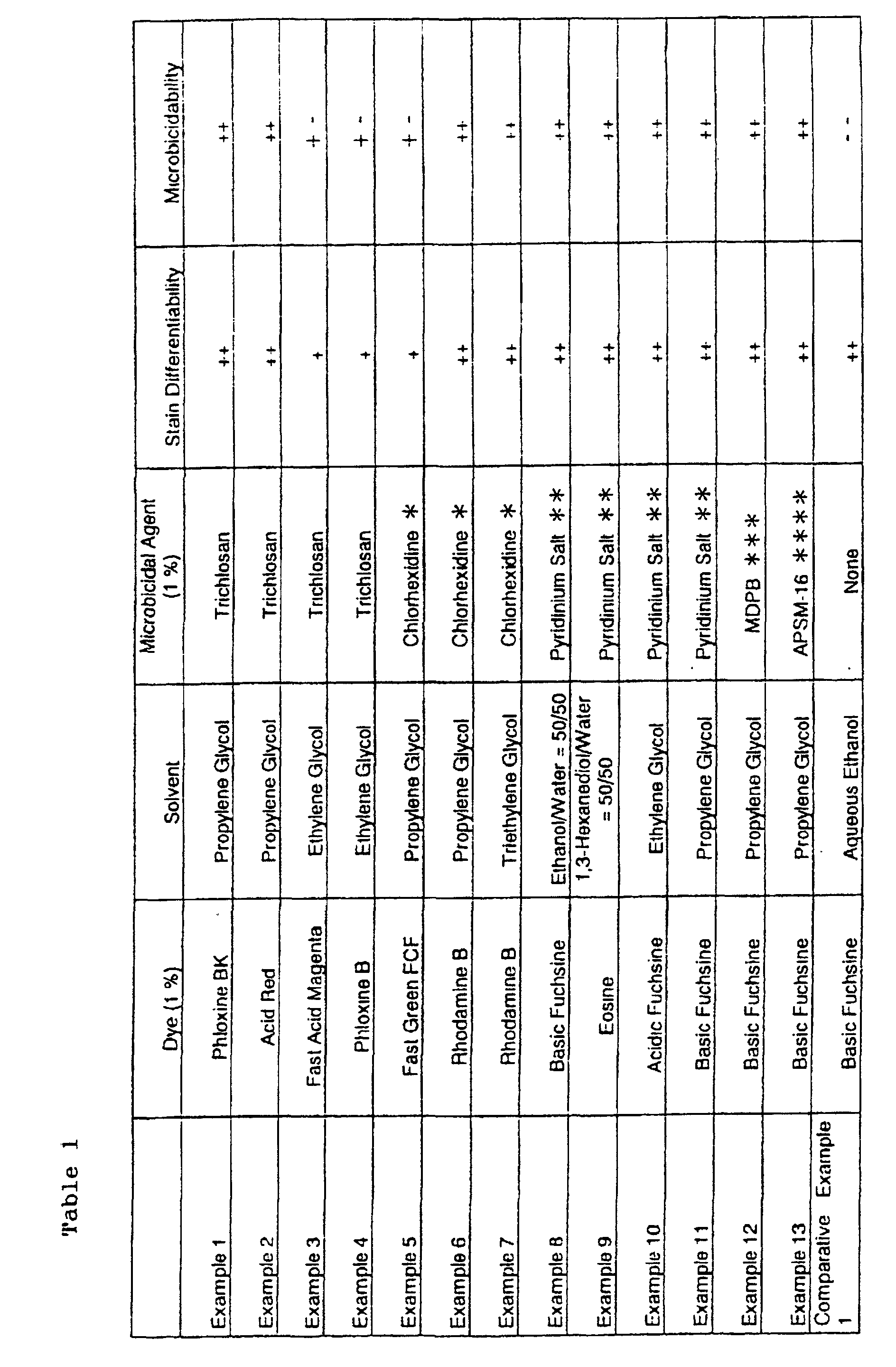

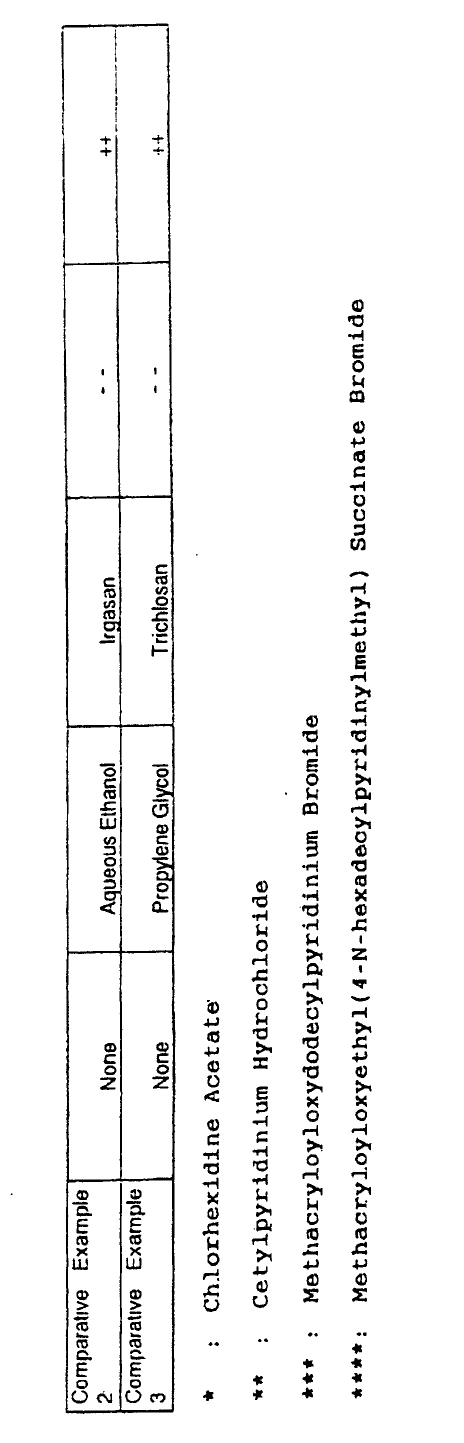

## Claims

1. An antimicrobial caries-detecting composition, which comprises water and/or a water-miscible solvent, a dye in a concentration from 0.1 to 2% by weight being capable of staining the caries-infected part of teeth, and at least an antimicrobial agent selected from the group consisting of cationic microbicides, biguanide-type microbicides and halogenated diphenyl ether-type microbicides.

2. The antimicrobial caries-detecting composition according to Claim 1, wherein said antimicrobial agent is at least one selected from the group consisting of trichlosan, irgasan, chlorhexidine and cetylpyridinium hydrochloride.

3. An antimicrobial caries-detecting composition, which comprises water and/or a water-miscible solvent, a dye in a concentration from 0.1 to 2% by weight being capable of staining the caries-infected part of teeth, and at least one polymerizable antimicrobial agent selected from the group consisting of compounds having a (meth)acryloyl or styrene group and a quaternary ammonium salt group in the molecule and represented by the following general formulae 1, 2, 3 and 4:
H₂C=C(R¹)-C(O)-X-R²-Y (1)
wherein
R¹= H or CH₃,
R²= alkylene group of C₂-C₁₂,
X = O, S, or NH,
Y = 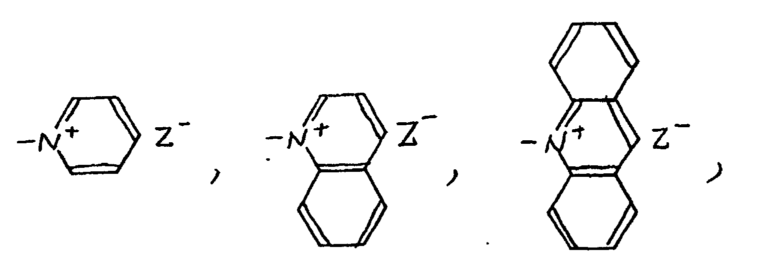 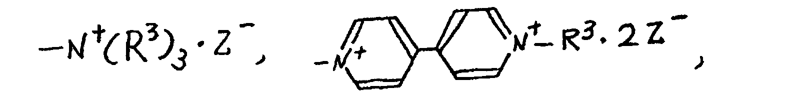 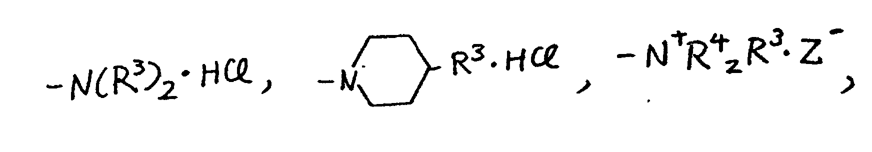 or 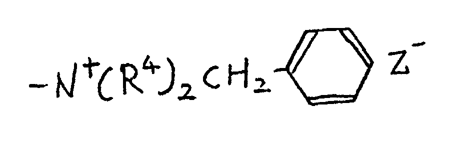 wherein
R³ = H or alkyl group of C₁-C₁₈;
R⁴ = CH₃, CH₂CH₃, or CH₂CH₂OH;
Z = F, Cl, Br, or I;
[H₂C=C(R⁵)-C(O)-X-R⁶-]₂-Y' (2)
wherein
R⁵ = H or CH₃,
R⁶ = alkylene group of C₂-C₁₂,
X = O, S, or NH,
Y' = 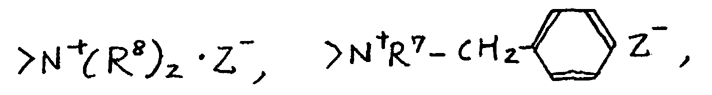 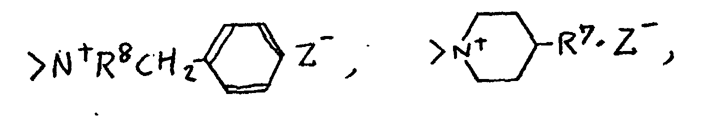 or 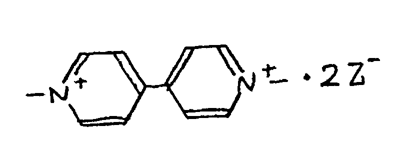 wherein
R⁷ = H or alkyl group of C₁-C₁₈;
R⁸ = CH₃, CH₂CH₃, or CH₂CH₂OH;
Z = F, Cl, Br, or I;
H₂C=C(R⁹)-C6H4-R¹⁰-Y (3)
wherein
R⁹ = H or CH₃,
R¹⁰ = alkylene group of C₂-C₁₂,
Y = the same as Y in formula (1); 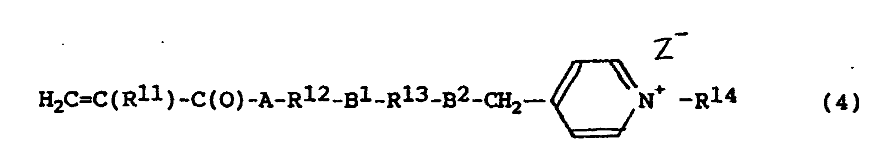 wherein
R¹¹ = H or CH₃,
R¹² = alkylene group of C₁-C₁₂,
R¹³ = alkylene group of C₁-C₁₂,
R¹⁴ = alkyl group of C₁₂-C₂₂,
A = O, S, or NH,
B¹, B² = the same or different groups selected from -CO-, -CO₂-, -O-, -S-, -OCONH- and -NHCO₂-,
Z = F, Cl, Br, or I.

## Patentansprüche

1. Antimikrobielle Karies-nachweisende Zusammensetzung, welche Wasser und/oder ein wassermischbares Lösungsmittel, einen Farbstoff in einer Konzentration von 0,1 bis 2 Gew.-%, der geeignet ist, den Karies-infizierten Teil der Zähne anzufärben, und mindestens ein antimikrobielles Mittel, ausgewählt aus der Gruppe, bestehend aus kationischen, mikrobiziden Mitteln, Biguanid-artigen, mikrobiziden Mitteln und halogenierten Diphenylether-artigen, mikrobiziden Mitteln, umfaßt.

2. Antimikrobielle Karies-nachweisende Zusammensetzung nach Anspruch 1, wobei das antimikrobielle Mittel mindestens eines, ausgewählt aus der Gruppe, bestehend aus Trichlosan, Irgasan, Chlorhexidin und Cetylpyridiniumhydrochlorid, ist.

3. Antimikrobielle Karies-nachweisende Zusammensetzung, welche Wasser und/oder ein wassermischbares Lösungsmittel, einen Farbstoff in einer Konzentration von 0,1 bis 2,0 Gew.-%, der geeignet ist, den Karies-infizierten Teil der Zähne anzufärben, und mindestens ein polymerisierbares, antimikrobielles Mittel, ausgewählt aus der Gruppe, bestehend aus Verbindungen mit einer (Meth)acryloyl- oder Styrolgruppe und einer quartären Ammoniumsalzgruppe im Molekül, und durch die folgenden, allgemeinen Formeln 1, 2, 3 und 4 dargestellt ist
H₂C=C(R¹)-C(O)-X-R²-Y (1)
worin
R¹ = H oder CH₃,
R² = Alkylenrest von C₂-C₁₂,
X = O, S oder NH,
Y = 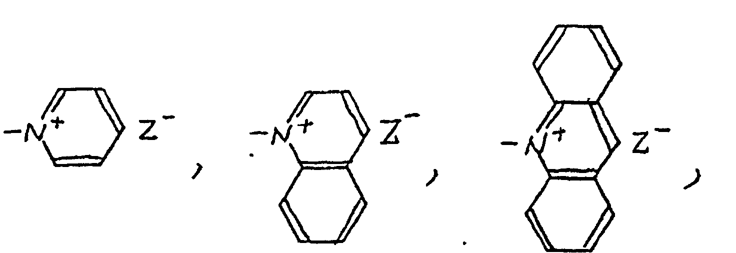 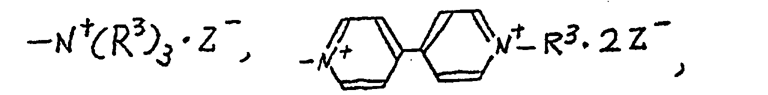 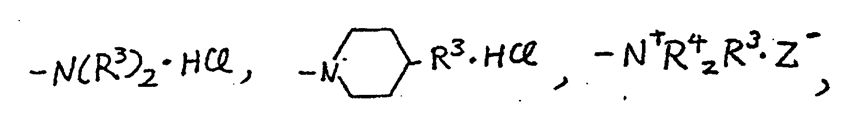 oder 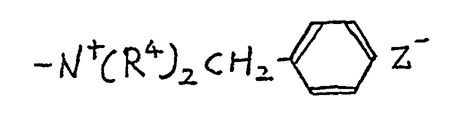 worin
R³ = H oder Alkylrest von C₁-C₁₈,
R⁴ = CH₃, CH₂CH₃ oder CH₂CH₂OH,
Z = F, Cl, Br oder I,
[H₂C=C(R⁵)-C(O)-X-R⁶-]₂-Y' (2)
worin
R⁵ = H oder CH₃,
R⁶ = Alkylenrest von C₂-C₁₂,
X = O, S oder NH,
Y' = 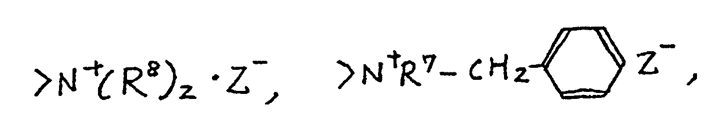 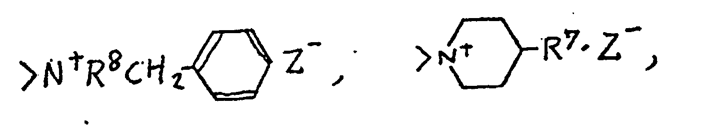 oder 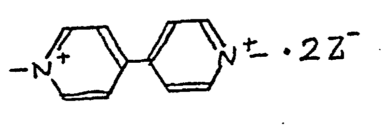 worin
R⁷ = H oder Alkylrest von C₁-C₁₈,
R⁸ = CH₃, CH₂CH₃ oder CH₂CH₂OH,
Z = F, Cl, Br oder I,
H₂C=C(R⁹)-C6H4-R¹⁰-Y (3)
worin
R⁹ = H oder CH₃,
R¹⁰ = Alkylenrest von C₂-C₁₂,
Y = das gleiche wie Y in Formel (1), 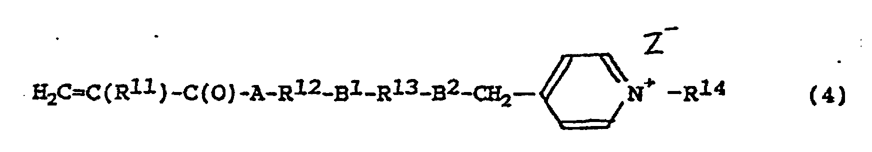 worin
R¹¹ = H oder CH₃,
R¹² = Alkylenrest von C₁-C₁₂,
R¹³ = Alkylenrest von C₁-C₁₂,
R¹⁴ = Alkylrest von C₁₂-C₂₂,
A = O, S oder NH,
B¹, B² = gleiche oder unterschiedliche Gruppen, ausgewählt aus -CO-,-CO₂-, -O-, -S-, -OCONH- und -NHCO₂-,
Z = F, Cl, Br oder I, umfaßt.

## Revendications

1. Composition antimicrobienne détectrice de caries, qui comprend de l'eau et/ou un solvant miscible à l'eau, un colorant à une concentration de 0,1 à 2 % en poids qui est capable de teinter la partie des dents infectée par des caries, et au moins un agent antimicrobien choisi dans le groupe consistant en des microbicides cationiques, des microbicides du type biguanide et des microbicides du type éther de diphényle halogéné.

2. Composition antimicrobienne détectrice de caries suivant la revendication 1, dans laquelle ledit agent anti-microbien est au moins un agent choisi dans le groupe consistant en le trichlosan, l'irgasan, la chlorhexidine et le chlorhydrate de cétylpyridinium.

3. Composition antimicrobienne détectrice de caries, qui comprend de l'eau et/ou un solvant miscible à l'eau, un colorant à une concentration de 0,1 à 2 % en poids qui est capable de teinter la partie des dents infectée par des caries, et au moins un agent antimicrobien polymérisable choisi dans le groupe consistant en des composés ayant un groupe (méth)acryloyle ou styrène et un groupe sel d'ammonium quaternaire dans la molécule et représentés par les formules générales 1, 2, 3 et 4 suivantes :
H₂C=C(R¹)-C (O)-X-R²-Y (1)
dans laquelle
R¹ représente H ou un groupe CH₃,
R² représente un groupe alkylène en C₂ à C₁₂,
X représente un atome de O, S ou un groupe NH
Y représente un groupe 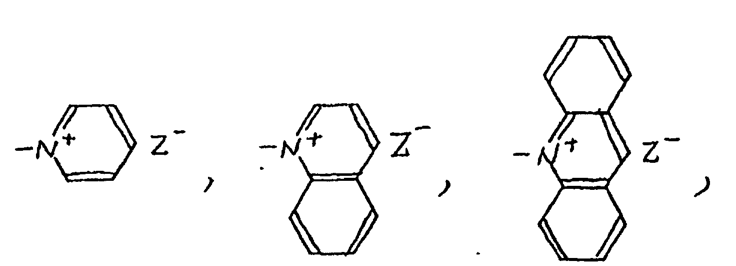 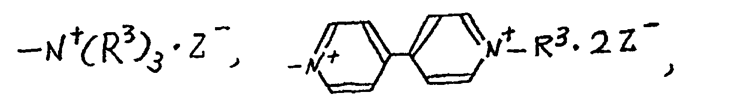 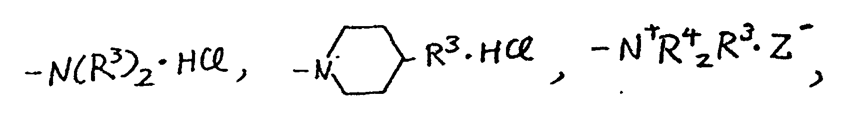 ou 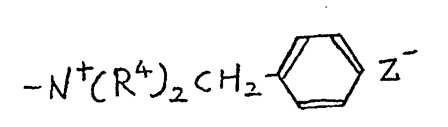 dans lequel
R³ représente H ou un groupe alkyle en C₁ à C₁₈ ;
R⁴ représente un groupe CH₃, CH₂CH₃ ou CH₂CH₂OH ;
Z représente un atome de F, Cl, Br ou I ;
[H₂C=C(R⁵)-C(O)-X-R⁶-]₂-Y' (2)
dans laquelle
R⁵ représente H ou un groupe CH₃,
R₆ représente un groupe alkylène en C₂ à C₁₂,
X représente un atome de O, S ou un groupe NH,
Y' représente un groupe 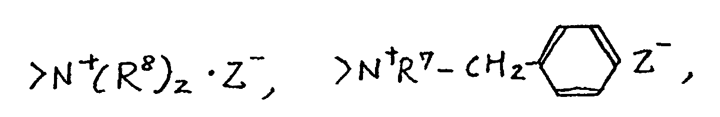 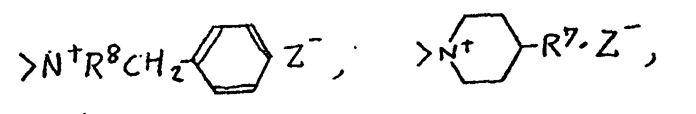 ou 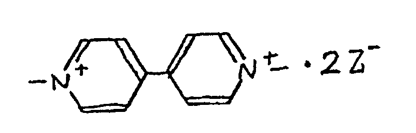 dans lequel
R⁷ représente H ou un groupe alkyle en C₁ à C₁₈ ;
R⁸ représente un groupe CH₃, CH₂CH₃ ou CH₂CH₂OH ;
Z représente un atome de F, Cl, Br ou I ;
H₂C=C(R⁹)-C₆H₄-R¹⁰-Y (3)
dans laquelle
R⁹ représente H ou un groupe CH₃,
R¹⁰ représente un groupe alkylène en C₁ à C₁₂,
Y est identique à Y dans la formule (1) ; 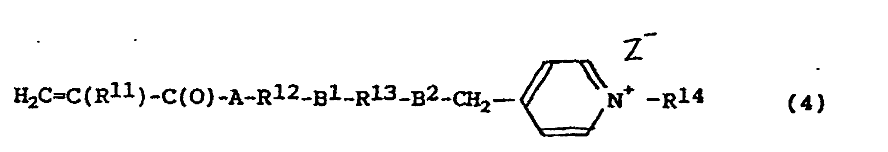 dans laquelle
R¹¹ représente H ou un groupe CH₃,
R¹² représente un groupe alkylène en C₁ à C₁₂,
R¹³ représente un groupe alkylène en C₁ à C₁₂,
R¹⁴ représente un groupe alkyle en C₁₂ à C₂₂,
A représente un atome de O, S ou un groupe NH,
B¹ et B² représentent des groupes, identiques ou différents, choisis entre des groupes -CO-, -CO₂-, -O-, -S-, -OCONH- et -NHCO₂-,
Z représente un atome de F, Cl, Br ou I.
